# EUROPÄISCHE PATENTSCHRIFT

(11) **EP 0 632 061 B1**
(45) Veröffentlichungstag und Bekanntmachung des Hinweises auf die Patenterteilung: **02.09.1998**
(21) Anmeldenummer: 94108424.6
(22) Anmeldetag: 01.06.1994
(51) Int. Cl.: C08F 8/46, C08F 10/00

(54) **Verfahren zur Herstellung von Polyisobutylbernsteinsäureanhydriden**
Process for producing polyisobutyl succinic anhydrides
Procédé de préparation d'anhydrides succiniques substituées par un reste polyisobutyle

(30) Priorität: 14.06.1993 DE 4319671
(43) Veröffentlichungstag der Anmeldung: 04.01.1995
(73) Patentinhaber: BASF Aktiengesellschaft, 67063 Ludwigshafen (DE)
(72) Erfinder: Greif, Norbert, Dr., D-67273 Bobenheim (DE); Oppenlaender, Knut, Dr., D-67061 Ludwigshafen (DE)

(56) Entgegenhaltungen:
- EP-A- 0 014 288
- EP-A- 0 355 895
- EP-A- 0 542 380
- GB-A- 944 136

## Beschreibung

Die vorliegende Erfindung betrifft ein verbessertes Verfahren zur Herstellung von Polyisobutylbernsteinsäureanhydriden mit einem durchschnittlichen molaren Verhältnis von Bernsteinsäureanhydridgruppen zu Polyisobutylgruppen von 1,05 : 1 bis 1,3 : 1.

Polyisobutylbernsteinsäureimide finden als sogenannte aschefreie Dispergatoren breite Anwendung im Schmierstoffbereich. In Motorölen für Otto- und Dieselmotoren sind sie in Mengen bis zu 10 Gew.-% enthalten und sollen hier die Agglomeration von Partikeln verhindern, die im allgemeinen als Schlammbildung bezeichnet wird. Die Dispergierwirkung dieser Zusätze ist für diese Anwendung die wichtigste Eigenschaft.

Polyisobutylbernsteinsäureimide werden im allgemeinen aus den entsprechenden Anhydriden hergestellt.

Polyisobutylbernsteinsäureanhydride wiederum werden üblicherweise aus Polyisobutenen und Maleinsäureanhydrid hergestellt. Zur Ausbeutesteigerung wird diese Reaktion häufig in Gegenwart von Chlor vorgenommen, was zur Folge hat, daß das Reaktionsprodukt chlorhaltig ist. Solche Produkte sind heute unerwünscht, da sie aufgrund ihrer Nebenbestandteile korrosionsfördernd sein können.

Eine rein thermische Umsetzung von Polyisobuten und Maleinsäureanhydrid kann zu chlorfreien Produkten führen. Jedoch sind dazu nach der Lehre der EP-A 457 599 relativ hohe Temperaturen von deutlich über 200°C in Verbindung mit Überdruck nötig, um wirtschaftliche Reaktionszeiten zu erzielen. Unter diesen Reaktionsbedingungen kommt es zu Teerbildung, die die Qualität des Produktes erniedrigt. Teerhaltige Produkte führen im Schmierstoff zur unerwünschten Bildung unlöslicher Bestandteile.

Die JP-A 89/79 163 lehrt ein Verfahren zur Herstellung von Alkenylbernsteinsäureanhydriden durch Umsetzung von Olefinen wie α-Olefinen mit höchstens 30 Kohlenstoffatomen mit Maleinsäureanhydrid in Gegenwart von Carbonsäuren als Katalysator.

Die EP-A 542 380 beschreibt ein Verfahren zur Herstellung von Polyalkenylderivaten aus ungesättigten Dicarbonsäuren in Gegenwart geringer Mengen von Sulfonsäuren als Katalysatoren.

Die GB-A 944 136 beschreibt die Herstellung von Alkenylbernsteinsäureanhydriden aus Polyolefinen und Maleinsäureanhydrid. Das eingesetzte Maleinsäureanhydrid kann 0,1 bis 0,3 Gew.-% Maleinsäure enthalten.

Für die Verwendung von Polyisobutylbernsteinsäureanhydriden als Schmierstoffadditiv ist ein gewisser Bismaleinierungsgrad erwünscht, d.h. daß die Produkte pro Polyisobuteneinheit im Durchschnitt mehr als eine Bernsteinsäureanhydridgruppe tragen.

Der Erfindung lag ein verbessertes Verfahren zur Herstellung von Polyisobutylbernsteinsäureanhydriden mit einem durchschnittlichen molaren Verhältnis von Bernsteinsäureanhydridgruppen zu Polyisobutylgruppen von 1,05 : 1 bis 1,3 : 1 als Aufgabe zugrunde, das es erlaubt, unter milden Reaktionsbedingungen zu Produkten zu gelangen, die weitgehend teerfrei anfallen.

Demgemäß wurde ein verbessertes Verfahren zur Herstellung von Polyisobutylbernsteinsäureanhydriden mit einem durchschnittlichen molaren Verhältnis von Bernsteinsäureanhydridgruppen zu Polyisobutylgruppen von 1,05 : 1 bis 1,3 : 1 gefunden, das dadurch gekennzeichnet ist, daß man Polyisobuten mit einem mittleren Molekulargewicht M_{w} von 600 - 5000 und einem Gehalt an endständigen Doppelbindungen von mindestens 70 % mit Maleinsäureanhydrid im Verhältnis von 1,05 : 1 bis 3 : 1 mol Maleinsäureanhydrid zu Polyisobuten bei 160 - 210°C in Gegenwart von 1 bis 10 Mol-%, bezogen auf Polyisobuten, einer Dicarbonsäure mit 2 bis 6 Kohlenstoffatomen umsetzt.

Die erfindungsgemäß zu verwendenden Polyisobutene sind bekannt (z.B. aus der EP-A 145 235) und weisen bei einem Molekulargewicht M_{w} von 600 - 5000, vorzugsweise 800 - 1200, einen Gehalt an endständigen Doppelbindungen von mindestens 70 %, vorzugsweise von 80 - 90 % auf. Diese endständige Doppelbindungen sind im Vergleich zu innenständigen Doppelbindungen bei Umsetzungen mit Maleinsäureanhydrid besonders reaktiv.

Zur Herstellung der Verfahrensprodukte ist es erforderlich, daß 1 Äquivalent Polyisobuten durchschnittlich mit mehr als 1 Äquivalent Maleinsäureanhydrid reagiert (sogenannte Bismaleinierung). Die En-Reaktion zwischen Polyisobuten und Maleinsäureanhydrid führt zunächst zu einem Produkt, das im Polyisobutylrest eine reaktive Doppelbindung trägt. Diese erlaubt eine weitere En-Reaktion mit Maleinsäureanhydrid zum bismaleinierten Produkt.

Die Ausgangsstoffe werden im molaren Verhältnis von 1,05 : 1 bis 3 : 1, vorzugsweise 1,1 : 1 bis 2 : 1 von Maleinsäureanhydrid zu Polyisobuten umgesetzt.

Die Reaktion wird in Gegenwart von 1 bis 10 Mol.-% einer Dicarbonsäure mit 2 bis 6 Kohlenstoffatomen vorgenommen. Bei den Säuren handelt es sich bevorzugt um aliphatische Verbindungen wie Oxalsäure, Maleinsäure, Fumarsäure und Adipinsäure oder Mischungen dieser Verbindungen. Bevorzugt sind Oxalsäure sowie Gemische aus Oxalsäure und Maleinsäure. Die Dicarbonsäuren können dem Reaktionsansatz zugesetzt werden. Maleinsäure kann aber auch durch Zugabe entsprechender Mengen Wasser aus Maleinsäureanhydrid unter den Reaktionsbedingungen gebildet werden. Die Mengen an Katalysator betragen 1 bis 10 Mol.-% bezogen auf Polyisobuten, vorzugsweise 3 bis 8 Mol.-%.

Die Reaktion wird bei 160 - 210°C vorgenommen. Im allgemeinen herrscht bei der Reaktion Normaldruck, es kann jedoch auch unter dem Eigendruck des Reaktionsgemisches oder bei erhöhtem Druck gearbeitet werden.

Die Reaktion kann in Substanz, aber auch in Gegenwart eines Lösungsmittels vorgenommen werden. Es sind hier besonders Kohlenwasserstoffe wie Naphtha und Petroleum (Siedebereich z.B. 170 - 210°C), weiterhin Ether wie Dimethyldiglykol und Diethyldiglykol zu nennen. Aber auch die Verfahrensprodukte selbst kommen als Lösungsmittel in Betracht. Üblicherweise beträgt die Menge des verwendeten Lösungsmittels 20 bis 50 Gew.-% bezogen auf den Reaktionsansatz.

Die Ausgangsstoffe können vor der Reaktion vermischt und bei der Reaktionstemperatur umgesetzt werden. In einer weiteren Ausführungsform wird nur ein Teil des Maleinsäureanhydrids vorgelegt und der verbleibende Teil wird der Reaktionsmischung bei Reaktionstemperatur so zugesetzt, daß immer eine homogene Phase im Reaktionsgefäß vorliegt.

Je nach der gewählten Reaktionstemperatur ist die Reaktion in der Regel nach 3 bis 10 Stunden beendet. In an sich bekannter Weise wird daraufhin auf das Verfahrensprodukt aufgearbeitet. Im allgemeinen werden dazu alle flüchtigen Bestandteile abdestilliert und der Destillationsrückstand wird isoliert.

Die nach dem erfindungsgemäßen Verfahren hergestellten Polyisobutylbernsteinsäureanhydride fallen weitgehend teerfrei an, was eine Weiterverarbeitung der Produkte ohne weitere Reinigungsmaßnahmen erlaubt.

Die nach dem erfindungsgemäßen Verfahren hergestellten Polyisobutylbernsteinsäureanhydride dienen als Zwischenprodukte für die Herstellung von Polyisobutylbernsteinsäureamiden und -imiden.

Diese Produkte werden in an sich bekannter Weise durch Umsetzung von Polyisobutylbernsteinsäureanhydriden mit einem primären oder sekundären Amin unter Abspaltung von Wasser erhalten.

Bei diesen Aminen kann es sich prinzipiell um alle zur Imidbildung geeigneten Verbindungen handeln wie Ammoniak, mono- und dialiphatische Amine, cycloaliphatische und aromatische Amine. Im Hinblick auf die Verwendung der Verfahrensprodukte als Zusatz zu Schmierstoffen sind jedoch Polyamine bevorzugt. Die Polyamine tragen vorzugsweise 2 bis 10 Stickstoffatome. Besonders bevorzugt sind solche Polyamine, die Alkylengruppen tragen wie Ethylen, 1,2-Propylen, 2,2-Dimethylpropylen, z.B. Ethylendiamin, Diethylentriamin, Triethylentetramin, Tetraethylenpentamin, Dipropylentriamin und Tripropylentetramin.

Die so erhältlichen Polyisobutylbernsteinsäureamide und -imide finden als Zusatz für Otto-, Wankel- und Dieselkraftstoffe Verwendung, insbesondere für Kraftstoffe in Ottomotoren, vorzugsweise in Mengen von 20 bis 300 ppm reiner Wirksubstanz bezogen auf den Kraftstoff. Sie können auch in Kombination mit anderen Dispergatoren wie Polyisobutylaminen verwendet werden.

Weiterhin eignen sie sich als Zusätze für Schmierstoffe, insbesondere für mineralische, teilsynthetische und vollsynthetische Motoröle. Sie werden in den Motorölen bevorzugt in Mengen von 1 bis 10 Gew.-%, bezogen auf das Öl, verwendet.

### Beispiele

### Allgemeine Herstellvorschrift

Polyisobuten (PIB) mit einem mittleren Molekulargewicht von 900 und einem Gehalt an endständigen Doppelbindungen von 85 % wurde mit Maleinsäureanhydrid (MSA) in Gegenwart von a Mol-% (bezogen auf PIB), einer Dicarbonsäure für 10 Stunden auf eine Temperatur T erhitzt. Nach Isolierung des Produktes wurde die Verseifungszahl VSZ, das molare Verhältnis von Bernsteinsäureanhydridgruppen zu Polyisobutylgruppen (Bismaleinierungsgrad) sowie in einigen Versuchen der Gehalt an Teer bestimmt (weitere Angaben zur Reaktion: s. Tabelle).

Zur Bestimmung des Teergehaltes wurde der Reaktionsrückstand in Hexan aufgenommen und der Teer wurde abfiltriert.

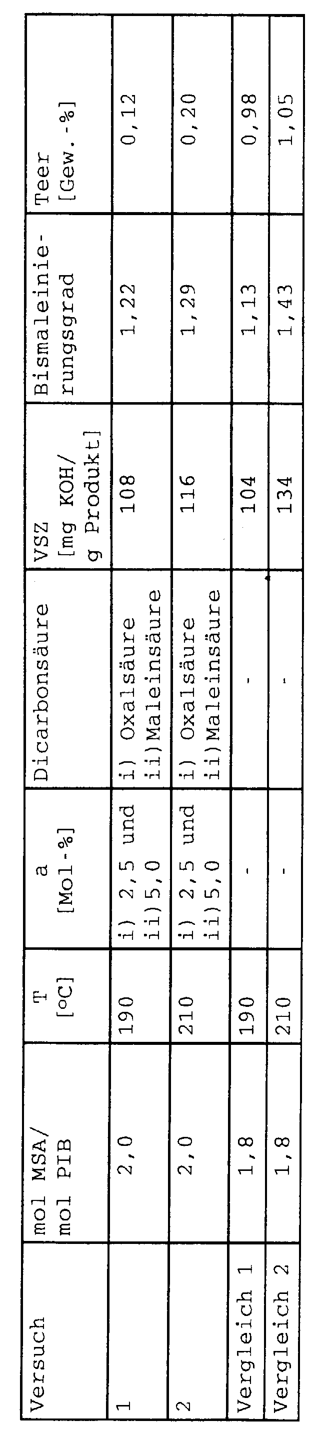

Die Versuche 1 - 2 und die Vergleichsversuche 1 und 2 zeigen, daß das erfindungsgemäße Verfahren zu einer dramatischen Reduzierung des Teergehaltes des Produktes führt.

## Patentansprüche

1. Verfahren zur Herstellung von Polyisobutylbernsteinsäureanhydriden mit einem durchschnittlichen molaren Verhältnis von Bernsteinsäureanhydridgruppen zu Polyisobutylgruppen von 1,05 : 1 bis 1,3 : 1, dadurch gekennzeichnet, daß man Polyisobuten mit einem mittleren Molekulargewicht M_{w} von 600 - 5000 und einem Gehalt an endständigen Doppelbindungen von mindestens 70 % mit Maleinsäureanhydrid im Verhältnis von 1,05 : 1 bis 3 : 1 mol Maleinsäureanhydrid zu Polyisobuten bei 160 - 210°C in Gegenwart von 1 bis 10 Mol-%, bezogen auf Polyisobuten, einer Dicarbonsäure mit 2 bis 6 Kohlenstoffatomen umsetzt.

2. Verfahren nach Anspruch 1, dadurch gekennzeichnet, daß das mittlere Molekulargewicht M_{w} des Polyisobutens 800 bis 1200 beträgt.

3. Verfahren nach Anspruch 1 oder 2, dadurch gekennzeichnet, daß der Gehalt an endständigen Doppelbindungen im Polyisobuten 80 - 90 % beträgt.

4. Verfahren nach Ansprüchen 1 bis 3, dadurch gekennzeichnet, daß man als Dicarbonsäure Oxalsäure verwendet.

## Claims

1. A process for the preparation of polyisobutylsuccinic anhydrides having an average molar ratio of succinic anhydride groups to polyisobutyl groups of from 1.05 : 1 to 1.3 : 1, wherein polyisobutene having an average molecular weight M_{w} of 600 - 5000 and containing at least 70% of terminal double bonds is reacted with maleic anhydride in a molar ratio of maleic anhydride to polyisobutene of from 1.05 : 1 to 3 : 1 at 160 - 210°C in the presence of from 1 to 10 mol%, based on polyisobutene, of a dicarboxylic acid of 2 to 6 carbon atoms.

2. A process as claimed in claim 1, wherein the average molecular weight M_{w} of the polyisobutene is from 800 to 1200.

3. A process as claimed in claim 1 or 2, wherein the content of terminal double bonds in the polyisobutene is 80 - 90%.

4. A process as claimed in any of claims 1 to 3, wherein the dicarboxylic acid used is oxalic acid.

## Revendications

1. Procédé de préparation d'anhydrides succiniques polyisobutylés ayant un rapport moyen en moles des groupements anhydride succinique aux groupements polyisobutyle allant de 1,05:1 à 1,3:1, caractérisé en ce que l'on fait réagir du polyisobutène ayant une masse molaire moyenne M_{w} de 600-5000 et une teneur en doubles liaisons terminales d'au moins 70% avec de l'anhydride maléique dans un rapport en moles de 1,05:1 à 3:1 de l'anhydride maléique au polyisobutène à 160-210°C en présence de 1-10% en moles, par rapport au polyisobutène, d'un acide dicarboxylique à 2-6 atomes de carbone.

2. Procédé selon la revendication 1, caractérisé en ce que la masse moléculaire moyenne M_{w} du polyisobutène s'élève à 800-1200.

3. Procédé selon la revendication 1 ou 2, caractérisé en ce que la teneur en doubles liaisons terminales du polyisobutène s'élève à 80-90%.

4. Procédé selon l'une quelconque des revendications 1 à 3, caractérisé en ce que l'on utilise de l'acide oxalique en tant qu'acide dicarboxylique.
